# EUROPÄISCHE PATENTANMELDUNG

(11) **EP 1 785 090 A1**
(43) Veröffentlichungstag der Anmeldung: **16.05.2007**
(21) Anmeldenummer: 05024477.1
(22) Anmeldetag: 10.11.2005
(51) Int. Cl.: A61B 5/15

(54) **Stechelement, Stechsystem und Verfahren zur Hautdetektion**

(71) Anmelder: F.HOFFMANN-LA ROCHE AG, 4070 Basel (CH); Roche Diagnostics GmbH, 68305 Mannheim (DE)
(72) Erfinder: Calasso, Irio Giuseppe, Dr., 6415 Arth (CH); Korner, Stephan, 6330 Cham (CH); Bründler, Oliver, 6043 Adligenswil (CH); Wahl, Hans-Peter, 79650 Schopfheim (DE); Glauser, Michael, 6343 Rotkreuz (CH)
(74) Vertreter: Pfiz, Thomas

(57) **Zusammenfassung**

Bei einem disposiblen Stechelement zur Entnahme von Körperflüssigkeit durch die Haut (22) mit einem Träger (12) und einem daran distal abstehenden, in einer Stechbewegung die Haut (22) einstechbaren Stechorgan (14), wird vorgeschlagen, dass an dem Träger (12) ein die Haut (22) im Bereich der Einstichstelle straffendes Vorspannmittel (16) angeordnet ist, welches ein bei der Stechbewegung seitlich von dem Stechorgan (14) auf die Haut (22) auftreffendes Kontaktglied (36) aufweist.

## Beschreibung

Die Erfindung betrifft ein Stechelement zur Gewinnung von Körperflüssigkeit durch bzw. über die Haut, insbesondere als Einwegteil für Blutzuckertests, mit einem Träger und einem starr daran abstehenden, in einer Stechbewegung in die Haut einstechbaren Stechorgan. Die Erfindung betrifft weiter ein Stechsystem zum Einsatz eines solchen vorzugsweise disposiblen Stechelements und ein Verfahren zur Hautdetektion für eine solche Probennahme.

Aus der WO 2005/096941 A1 ist ein System zur Blutentnahme mittels eines eine trägergebundene Sammeleinheit aufweisenden Stechelements bekannt, bei dem durch einen geräteseitig gelagerten Druckring auf einen dagegen gedrückten Finger ein Druck ausgeübt wird, um in der komprimierten Fingerpartie ausreichend Blut bereit zu stellen und anschließend durch Druckreduzierung das Austreten von Blut zu vermeiden. Zur Vermeidung einer Kontamination mit Blut und für die ausreichende Erhöhung des Körperinnendrucks muss der Druckring einen Durchmesser in der Größe der Fingerbeere besitzen, wodurch das Körperteil im Einstechbereich ausgewölbt wird. Durch die beim Stechvorschub auftreffende Nadel erfolgt dann eine Hauteindellung nach innen, bis schließlich abhängig vom Hauttyp und der Hautdicke des Probanden ein Stichkanal erzeugt wird.

Ausgehend hiervon liegt der Erfindung die Aufgabe zugrunde, die im Stand der Technik bekannten Systeme weiter zu entwickeln und eine gattungsgemäße Anordnung und ein Verfahren insbesondere im Sinne einer definierten Einstechtiefe zu optimieren, wobei ein Erfindungsziel auch in einer vereinfachten und hygienisch vorteilhaften Systemauslegung besteht.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, durch einen Hautstraffer als integraler Bestandteil des Stechelements die Haut im Einwirkungsbereich des Stechorgans einzudrücken. Dementsprechend wird erfindungsgemäß vorgeschlagen, dass an dem Träger ein die Haut im Bereich der Einstichstelle straffendes Vorspannmittel angebracht ist, welches ein bei der Stechbewegung auf die Haut aufstoßendes und dabei entgegen dem Stechorgan zurück bewegliches Kontaktglied aufweist. Das Kontaktglied sorgt damit für eine konkave Hauteinwölbung an der Einstichstelle, so dass durch das auftreffende Stechorgan keine Hautverdrängung mehr erfolgt und sofort der Stichkanal erzeugt wird. Die Freigabe des Stechorgans erfolgt unter relativer Rückbewegung des an der Haut anliegenden Kontaktglieds, wobei sich durch die Hautstraffung direkt an der Einstichstelle der Einfluss hautspezifischer Schwankungen auf die Stechtiefe weitgehend eliminieren lässt. Zudem kann eine Gerätekontamination mit Körperflüssigkeit durch die integrale Anordnung des Kontaktglieds an dem vorzugsweise disposiblen Stechelement zuverlässig vermieden werden.

Vorteilhafterweise ist das Kontaktglied im Ausgangszustand dem Stechorgan in Stechrichtung vorgelagert und bei der Stechbewegung auf der Haut gegen den Träger rückbeweglich abgestützt. Auf diese Weise wird durch eine einfache Vorschubbewegung das Eindringen des Stechorgans in vorgespannte Haut sichergestellt. Grundsätzlich könnte es auch genügen, wenn sich das Kontaktglied im Wesentlichen auf gleicher Höhe mit dem Stechorgan befindet, so dass zumindest beim Eintritt in die blutgebene Hautzone die Hautstraffung erfolgt ist.

Eine weitere vorteilhafte Ausführung sieht vor, dass das Vorspannmittel ein das Kontaktglied mit dem Träger verbindendes, elastisch und/oder plastisch komprimierbares und/oder durch Reibschluss gehaltenes Kopplungsteil aufweist. Damit wird eine weitere Vorwärtsbewegung des Stechelements ermöglich, während sich das Kontaktglied bereits in Anlage mit der Haut befindet. Hierbei ist es auch herstellungstechnisch vorteilhaft, wenn das Kopplungsteil durch einen an mindestens einer Biegestelle abbiegbaren bzw. knickbaren Faltarm gebildet ist. Ein zusätzlicher Federweg kann dadurch bereitgestellt werden, dass das Kontaktglied über ein Federelement an dem Träger abgestützt ist.

Um bei der Rückbewegung in eine Sammelposition die Kontaktkraft weitgehend aufzuheben, ist es von Vorteil, wenn das Kontaktglied in einer gegenüber dem Stechorgan proximal zurückgestellten Rückstellposition durch einen Sperrmechanismus, insbesondere eine Klinke selbsttätig arretierbar ist.

Für die Blutgewinnung ist es vorteilhaft, wenn das Kontaktglied beim Zurückziehen des Trägers unter Verringerung oder Aufhebung der Anpresskraft an der Haut mitbeweglich ist.

Herstellungstechnisch und in funktionaler Hinsicht ist es von Vorteil, wenn das Vorspannmittel als integraler Bestandteil vorzugsweise einstückig an dem Träger angebracht ist. Eine weitere Verbesserung sieht vor, dass das Stechelement aus einem flachen Substrat gebildet, insbesondere geätzt ist, und dass das Vorspannmittel als Substratteil in der Substratebene angeordnet oder aus dieser herausgebogen ist.

Gemäß einer weiteren vorteilhaften Ausgestaltung besitzt das Kontaktglied eine seitlich neben dem Stechorgan gegen die Haut andrückbare punkt- oder linienförmige Randkontur aufweist. Dies sollte so nahe wie möglich an der Einstichstelle erfolgen, ohne jedoch mit dem Stechorgan zu kollidieren. Entsprechend ist es vorteilhaft, wenn das Stechorgan in einem seitlichen Abstand von weniger als 3 mm, vorzugsweise 1 bis 2 mm von dem Kontaktglied in die Haut einstechbar ist.

Zur Stechtiefenregulierung ist es günstig, wenn an dem Träger ein die Einstechtiefe des Stechorgans begrenzender Anschlag einstückig angebracht bzw. angeformt ist, welcher eine bei der Stechbewegung in definiertem proximalem Abstand zu dem Stechorgan anschlagende Hautkontaktfläche aufweist.

Vorteilhafterweise ist der Anschlag zur Stechtiefenregulierung vorzugsweise durch Biegeverformung oder veränderliche Raststellen in seiner an dem Basisteil vorspringenden Länge in Stechrichtung längenveränderlich.

Das Stechelement kann als einfache Lanzette ausgebildet sein, während für ein gleichzeitiges Sammeln von Körperflüssigkeit eine vorzugsweise kapillaraktive Aufnahmestruktur, die in den Bereich des Stechorgans reicht, von Vorteil ist.

Gegenstand der Erfindung ist auch ein Stechsystem zur Gewinnung von Körperflüssigkeit durch bzw. über die Haut mit einem Stechantrieb und einem damit in einer Stechbewegung vorwärts und zurück bewegbaren erfindungsgemäßen Stechelement.

Eine besonders bevorzugte Variante sieht einen Positionsdetektor zur Erfassung der Position der gestrafften Haut im Zuge der Stechbewegung vor. Auf diese Weise ist eine sehr genaue Stechtiefenbestimmung möglich, ohne dass ein "Leerweg" des Stechorgans aufgrund von Hauteindellung zu Fehlern führt.

Hierbei ist es vorteilhaft, wenn der Positionsdetektor die Position der durch das Vorspannmittel gestrafften Haut über das Kontaktglied und/oder das Stechorgan als Fühler abtastet, und wenn der Positionsdetektor eine Kapazitäts- oder Leitfähigkeitsänderung oder eine Kraftänderung beim Abtasten der Haut erfasst.

Gemäß einer weiteren vorteilhaften Ausführung ist das Stechelement an eine vorzugsweise inkremental arbeitende Wegmesseinheit zur Erfassung der Relativposition von Kontaktglied und Stechorgan angeschlossen.

Für einen definierten und damit möglichst schmerzarmen Stechvorgang ist es von Vorteil, wenn der Stechantrieb eine Einrichtung zur Einstellung der Stechtiefe des Stechorgans bezüglich einer erfassten Referenzposition der gestrafften Haut aufweist.

In verfahrensmäßiger Hinsicht wird die eingangs genannte Aufgabe im Sinne einer Hautdetektion für die Probennahme von Körperflüssigkeit, wobei die Position der Haut bezüglich einer Bewegungsachse eines Stechelements erfasst wird, dadurch gelöst, dass die Haut durch ein Vorspannmittel des Stechelements bei der Positionserfassung lokal gestrafft wird.

Um die Stechbewegung mit hoher Dynamik ausführen zu können, ist es vorteilhaft, wenn das Stechelement nach der Positionserfassung und vor einer Stechbewegung in eine von der Haut zurückgezogene Startstellung bewegt wird. In diesem Zusammenhang ist es auch günstig, wenn die Stechbewegung des Stechelements zur Einstellung einer definierten Stechtiefe nach Maßgabe der erfassten Hautposition gesteuert wird.

Im Folgenden wird die Erfindung anhand der in der Zeichnung schematisch dargestellten Ausführungsbeispiele näher erläutert. Es zeigen
- Fig. 1: ein Blutzuckermessgerät mit einem zur Hautstraffung an der Stechstelle ausgebildeten Stechelement in einer schematisch vereinfachten Darstellung;
- Fig.2 bis 4: weitere Ausführungsformen eines Stechelements mit Hautstraffer;
- Fig. 5: das Stechelement nach Fig. 1 in verschiedenen Positionen bei der Stechbewegung;
- Fig. 6: eine Ausführungsform eines Stechelements mit einrastbarem Hautstraffer in einer Seitenansicht;
- Fig. 7: eine weitere Ausführungsform eines Einrastmechnismus in ausschnittsweiser Darstellung;
- Fig. 8: einen Hautsensor an einem Stechelement in ausschnittsweiser Ansicht im Ausgangszustand und Stechzustand;
- Fig. 9: und 10 Stechelemente mit verstellbarem Abstandshalter zur Stechtiefenregulierung in schaubildlicher Darstellung.

Die in der Zeichnung dargestellten Stechelemente 10 besitzen einen Träger bzw. ein Basisteil 12 als Halter, ein an dem Halter in Stechrichtung (distal) abstehendes, als Spitze ausgebildetes Stechorgan 14 und ein neben dem Stechorgan auf die Haut aufsetzbares Vorspannmittel 16 als Hautstraffer.

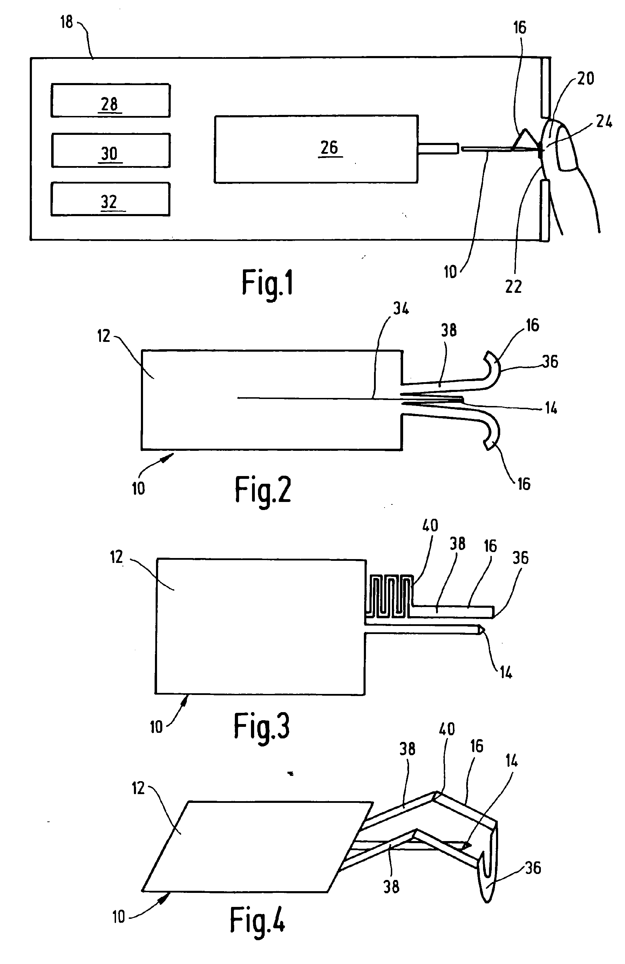

Gemäß Fig. 1 lassen sich solche Stechelemente 10 als Einmalartikel (so genanntes "Disposable") in ein Handgerät 18 einsetzen, um in einer Stechbewegung Körperflüssigkeit, d.h. Blut, gegebenenfalls auch Gewebeflüssigkeit speziell für Blutzucker-Selbsttests zu gewinnen. Hierbei wird von dem Benutzer ein Körperteil, insbesondere die Fingerbeere 20 auf eine Geräteöffnung aufgesetzt. Sodann wird in einer vorwärts und zurück verlaufenden Stechbewegung gegen die in der Geräteöffnung liegende Hautpartie 22 mittels des Stechorgans 14 an einer Einstichstelle eine geringe Blutmenge gewonnen und vorzugsweise im Gerät 18 analysiert.

Zu diesem Zweck weist das Gerät 18 einen das eingesetzte Stechelement 10 bewegenden Stechantrieb 26, einen Hautdetektor 28, eine damit zusammenwirkende Einrichtung zur Stechtiefeneinstellung 30 und weitere Gerätebaugruppen wie eine Analyseeinheit 32 auf. Das auf dem Testelement 10 gesammelte Blut kann damit für eine Blutzuckerbestimmung vor Ort in einem automatischen Messablauf verwertet werden. Anschließend wird das gebrauchte Testelement entsorgt und vorzugsweise aus einem Gerätemagazin ein neues Testelement bereitgestellt, so dass eine möglichst hygienische Handhabung gewährleistet ist.

Das in Fig. 2 gezeigte Testelement 10 ist aus einem flachen Substrat, beispielsweise Edelstahlblech geätzt, wobei das Stechorgan 14 und das Vorspannmittel 16 als in der Ebene des Substrats liegende integrale Bestandteile zusammen mit dem Basisteil 12 freigeätzt sind. Auf diese Weise können einstückige Stechelemente in einem für die Massenfertigung geeigneten einheitlichen Verfahrensablauf hergestellt werden. Um zusätzlich eine Sammelfunktion für die an der Einstichstelle gewonnene Körperflüssigkeit zu integrieren, ist ein längsseitig halboffener Kapillarkanal 34 über das Basisteil 12 bis in den Bereich des Stechorgans 14 geführt.

Das Vorspannmittel 16 ist durch ein bei der Stechbewegung seitlich vor dem Stechorgan 14 auf die Haut auftreffendes Kontaktglied 36 und ein das Kontaktglied mit dem Träger 12 verbindendes Kopplungsteil 38 gebildet. Auf diese Weise wird das Vorspannmittel 16 als integrale Struktur mit dem Träger 12 mitbewegt. Das Kontaktglied 36 wird bei der Vorwärtsbewegung des Stechelements 10 vor dessen Hautkontakt mit einer punkt- oder linienförmigen Randkontur auf die Haut 22 aufgesetzt. Beim weiteren Vorschub wird das Kopplungsteil 38 durch das in Anlage auf der Haut relativ zu dem Stechorgan 14 zurückbewegte Kontaktglied 36 elastisch und/oder plastisch in seiner distalen Länge verformt, so dass das Stechelement 14 in die zuvor gestraffte Haut einsticht, wie es nachstehend näher erläutert wird.

Bei dem in Fig. 3 gezeigten Ausführungsbeispiel wird das Kontaktglied 36 ebenfalls in geringem seitlichem Abstand und in Stechrichtung vor dem Stechorgan 14 gehalten, wobei ein Federelement 40 eine begrenzte Rückbewegung gegen das Basisteil 12 beim Stechvorschub erlaubt. Um eine Hautstraffung nahe der Einstichstelle zu erreichen, beträgt der seitliche Abstand zwischen dem Kontaktglied 36 und dem Stechorgan 14 in der Wirkstellung weniger als 3 mm, vorzugsweise 1 bis 2 mm.

Während bei den Ausführungen nach Fig. 2 und 3 das Vorspannmittel 16 in der Substratebene angeordnet ist, sieht das Ausführungsbeispiel nach Fig. 4 einen Doppel-Faltarm 38 als Kopplungsteil vor, welcher an einer aus der Substratebene heraus abknickbaren Biegestelle 40 eine distale Verkürzung gegenüber dem Stechorgan 14 erlaubt.

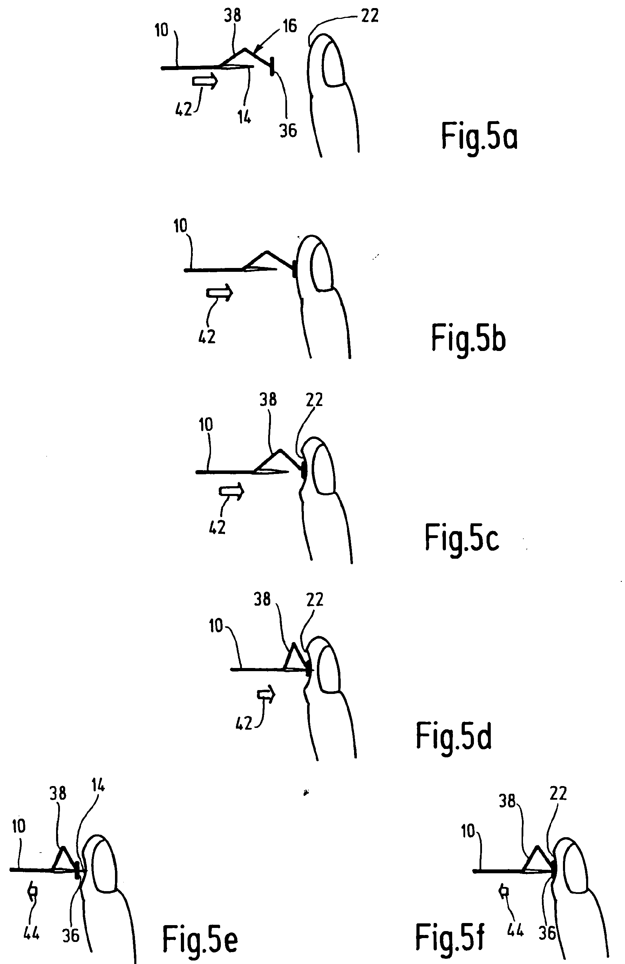

Fig. 5 veranschaulicht die Hautstraffung beim Stech- und Sammelvorgang in verschiedenen Bewegungsstadien. Bei der Vorwärtsbewegung (Pfeil 42) befindet sich das Kontaktglied 36 des Vorspannmittels bzw. Hautstraffers 16 vor dem Stechorgan (Fig. 5a) und trifft somit nahe der vorgesehenen Einstichstelle auf die Haut 22 auf (Fig. 5b). Beim weiteren Vorschub wird die Haut 22 mit definierter Kraft verdrängt, wobei der Faltarm 38 komprimiert wird (Fig. 5c), bis schließlich der Nadeleinstich in die vorgespannte Haut 22 erfolgt (Fig. 5d).

Nach Erreichen der vorgesehenen und gegenüber der gestrafften Haut definierten Einstechtiefe wird das Stechelement in einer Rückbewegung (Pfeil 44) auf eine weniger tief eingestochene Sammelposition zurückgezogen. Sofern der Faltarm 38 zuvor plastisch verformt wurde, folgt das Kontaktglied 36 der Rückbewegung, wie es in Fig. 5e gezeigt ist. Dabei wird die Andrückkraft weitgehend aufgehoben, und die Haut 22 im Bereich der Einstichstelle relaxiert. Dadurch wird weniger Körperflüssigkeit in der betreffenden Hautpartie verdrängt, und es kann in kurzer Zeit eine ausreichende Flüssigkeitsmenge (Mikroliter oder weniger) gewonnen werden. Bei der in Fig. 5f gezeigten Alternative wurde der Faltarm 38 beim Einstechen nicht plastisch verformt, sondern nur elastisch um eine bestimmte Länge zusammengedrückt. Beim Zurückziehen verbleibt somit das Kontaktglied 36 auf der Haut 22, während die Rückstellkraft des Faltarms 38 sich verringert. Damit ist auch hier die Verdrängung der Körperflüssigkeit im Körperteil kleiner als bei der maximalen Einstechtiefe.

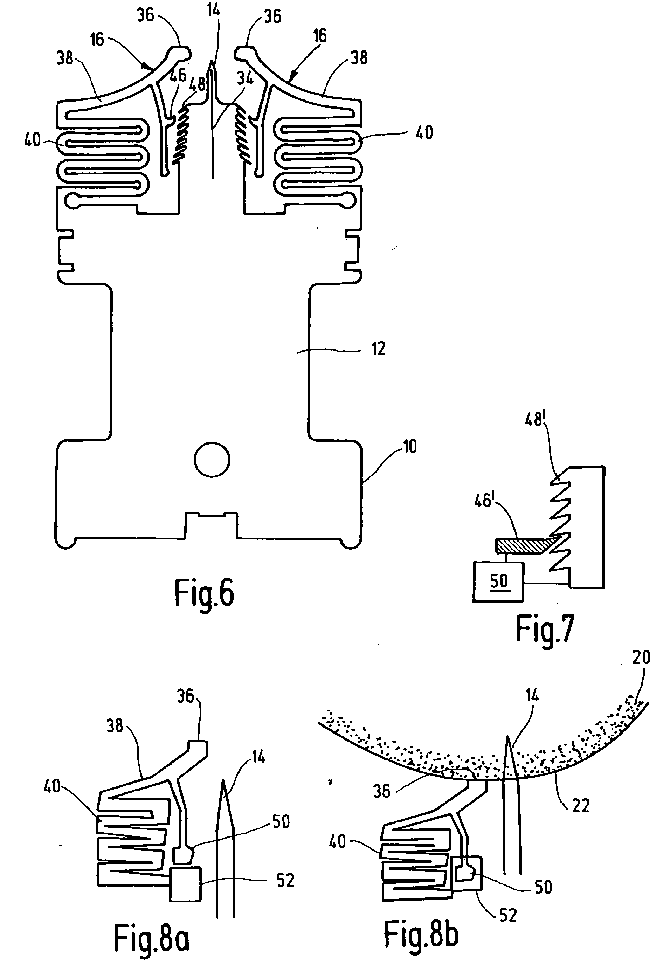

Bei der in Fig. 6 gezeigten Ausführungsform sind vorstehend bereits beschriebene Teile mit den gleichen Bezugszeichen versehen. Zusätzlich ist hier an dem Kopplungsteil 38 eine Sperrklinke 46 angeformt, welche in eine Verzahnung 48 an dem Basisteil 12 zur Sicherung der erreichten Rückstellposition des Kontaktglieds 36 einhakbar ist. Dadurch wird bei maximalem Vorschub des Stechelements 10 und entsprechender elastischer Rückstellung des Vorspannmittels 16 in Anlage mit der Haut 22 eine Arretierung erreicht, so dass ähnlich der Ausführung nach Fig. 5e der komprimierte Vorspanner 16 bei der Rückbewegung in die Sammelposition nicht mehr gegen die Haut 22 andrückt.

Entsprechend Fig. 7 ist es auch denkbar, dass eine geräteseitige Rastklinke 46' in eine Verzahnung 48' des Vorspannmittels 16 richtungsabhängig arretierend eingreift. Grundsätzlich ist es mit einer solchen Anordnung auch möglich, über eine Wegmesseinheit 50 die Anzahl der Einrastklicks (beispielsweise mittels eines elektrischen Impuls-Zählers) zu erfassen und auf diese Weise die Relativverschiebung zwischen Kontaktglied 36 und Stechorgan 14 zu ermitteln und im Sinne einer Stechtiefenbestimmung auszuwerten.

Fig. 8 veranschaulicht eine Ausführungsform, bei der mittels des Hautdetektors 28 über das Kontaktglied 36 die Position der gestrafften Haut in Stechrichtung kapazitiv erfasst wird. Hierfür ist an dem Kopplungsteil 38 ein Elektrodenarm 50 angeordnet, welcher mit einer gerätefesten Gegenelektrode 52 als wegsensitive Kondensatoranordnung zusammenwirkt. Wie aus Fig. 8 zu ersehen, wird in der Vorschubstellung bei maximaler Hautstraffung die maximale Kapazität erreicht. Daraus lässt sich auch die Relativverschiebung zu dem Stechorgan 14 und damit dessen tatsächliche Einstechtiefe bestimmen, beispielsweise indem durch eine Kalibrierung empirische Vergleichswerte gewonnen werden. Die Stechtiefenerfassung kann noch während der Vorwärtsbewegung als Eingangssignal für die Einrichtung zur Stechtiefeneinstellung 30 benutzt werden, welche den Stechantrieb 26 entsprechend steuert bzw. regelt.

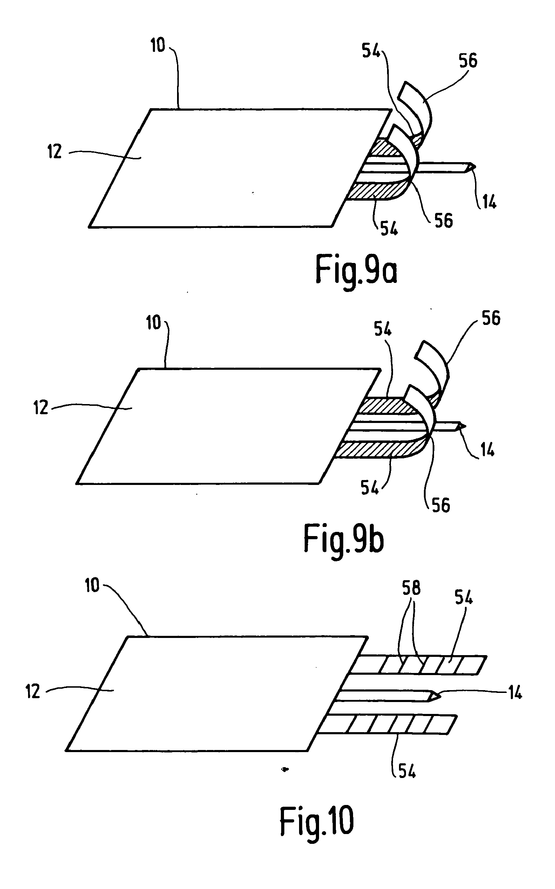

In Fig. 9 und 10 sind Ausführungsformen eines Stechelements 10 mit längenverstellbaren Anschlägen gezeigt, welche eine vorgegebene Stechtiefe des Stechorgans 14 in dem Körperteil definieren. Diese Anschläge 54 sind als integrierte Strukturen an dem Stechelement 10 angeformt und mit einer proximal hinter dem Stechorgan 14 liegenden Anschlagfläche 56 versehen. Wie aus Fig. 9a und 9b ersichtlich, ist die Anschlagposition gegenüber dem Stechelement 14 durch eine Biegeverformung, die im Gerät 18 vor dem Stechen ausgeführt wird, variabel, so dass entsprechend eine an das Körperteil angepasste Stechtiefe fest an dem Stechelement voreinstellbar ist. Damit kann beispielsweise unterschiedlichen Haut-Typen Rechnung getragen werden, um zuverlässig und zugleich schmerzarm die Blut gebende Zone zu erreichen.

In der Ausführung nach Fig. 10 sind an den Anschlägen 54 eine Mehrzahl von Knickstellen 58 vorgeformt, so dass die Biegeanpassung der Anschlagposition vereinfacht wird. Alternativ kann auch eine Längenanpassung durch Abbrechen von Segmenten erreicht werden. Die Einstellung wird im Gerät 18 an einem einzigen Teil, d.h. dem Stechelement 10 vorgenommen, so dass nicht mehrere Baueinheiten eingestellt werden müssen. Denkbar sind auch Varianten mit einer einrastbaren Verzahnung ähnlich der zu Fig. 6 beschriebenen Rückstellsicherung.

## Patentansprüche

1. Stechelement zur Gewinnung von Körperflüssigkeit durch die Haut (22), insbesondere als Einwegteil für Blutzuckertests, mit einem Träger (12) und einem starr daran abstehenden, in einer Stechbewegung in die Haut (22) einstechbaren Stechorgan (14), **dadurch gekennzeichnet, dass** an dem Träger (12) ein die Haut (22) im Bereich der Einstichstelle straffendes Vorspannmittel (16) angebracht ist, welches ein bei der Stechbewegung auf die Haut (22) aufstoßendes und dabei entgegen dem Stechorgan (14) zurück bewegliches Kontaktglied (36) aufweist.

2. Stechelement nach Anspruch 1, **dadurch gekennzeichnet, dass** das Kontaktglied (36) im Ausgangszustand dem Stechorgan (14) in Stechrichtung seitlich vorgelagert ist und bei der Stechbewegung in Kontakt mit der Haut gegen den Träger (12) rückstellbar ist.

3. Stechelement nach Anspruch 1 oder 2, **dadurch gekennzeichnet, dass** das Vorspannmittel (16) ein das Kontaktglied (36) mit dem Träger (12) verbindendes, elastisch und/oder plastisch komprimierbares und/oder durch Reibschluss gehaltenes Kopplungsteil (38) aufweist.

4. Stechelement nach Anspruch 3, **dadurch gekennzeichnet, dass** das Kopplungsteil (38) durch einen an mindestens einer Biegestelle (40) abbiegbaren Faltarm gebildet ist.

5. Stechelement nach einem der Ansprüche 1 bis 4, **dadurch gekennzeichnet, dass** das Kontaktglied (36) über ein Federelement (40) an dem Träger (12) abgestützt ist.

6. Stechelement nach einem der Ansprüche 1 bis 5, **dadurch gekennzeichnet, dass** das Kontaktglied (36) in einer gegenüber dem Stechorgan (14) proximal zurückgestellten Rückstellposition durch einen Sperrmechanismus (46,48), insbesondere eine Klinke arretierbar ist.

7. Stechelement nach einem der Ansprüche 1 bis 6, **dadurch gekennzeichnet, dass** das Kontaktglied (36) beim Zurückziehen des Trägers (12) unter Verringerung oder Aufhebung der Anpresskraft an der Haut (22) mitbeweglich ist.

8. Stechelement nach einem der Ansprüche 1 bis 7, **dadurch gekennzeichnet, dass** das Vorspannmittel (16) als integraler Bestandteil vorzugsweise einstückig an dem Träger (12) angebracht ist.

9. Stechelement nach einem der Ansprüche 1 bis 8, **dadurch gekennzeichnet, dass** es aus einem flachen Substrat gebildet, insbesondere geätzt ist, und dass das Vorspannmittel (16) als Substratteil in der Substratebene angeordnet oder aus dieser herausgebogen ist.

10. Stechelement nach einem der Ansprüche 1 bis 9, **dadurch gekennzeichnet, dass** das Kontaktglied (36) eine seitlich nahe dem Stechorgan (14) gegen die Haut (22) andrückbare punkt- oder linienförmige Randkontur aufweist.

11. Stechelement nach einem der Ansprüche 1 bis 10, **dadurch gekennzeichnet, dass** das Stechorgan (14) in einem seitlichen Abstand von weniger als 3 mm, vorzugsweise 1 bis 2 mm von dem Kontaktglied (36) in die Haut (22) einstechbar ist.

12. Stechelement zur Entnahme von Körperflüssigkeit durch die Haut (22), insbesondere nach einem der vorstehenden Ansprüche, mit einem Träger (12) und einem starr daran abstehenden, in einer Stechbewegung in die Haut (22) einstechbaren Stechorgan (14), **dadurch gekennzeichnet, dass** an dem Träger (12) ein die Einstechtiefe des Stechorgans (14) begrenzender Anschlag (54) einstückig angebracht ist, welcher eine bei der Stechbewegung in definiertem proximalem Abstand zu dem Stechorgan anschlagende Hautkontaktfläche aufweist.

13. Stechelement nach einem der Ansprüche 1 bis 12, **dadurch gekennzeichnet, dass** der Anschlag (54) zur Stechtiefenregulierung vorzugsweise durch Biegeverformung oder veränderliche Raststellen in seiner an dem Basisteil vorspringenden Länge veränderlich ist.

14. Stechelement nach einem der Ansprüche 1 bis 12, **gekennzeichnet durch** eine vorzugsweise kapillaraktive Aufnahmestruktur (34) für die Körperflüssigkeit.

15. Stechsystem zur Gewinnung von Körperflüssigkeit durch die Haut (22) mit einem Stechantrieb (26) und einem damit in einer Stechbewegung vorwärts und zurück bewegbaren Stechelement (10) nach einem der vorhergehenden Ansprüche.

16. Stechsystem nach Anspruch 15, **gekennzeichnet durch** einen Positionsdetektor (28) zur Erfassung der Position der gestrafften Haut (22) im Zuge der Stechbewegung des Stechelements (10)..

17. Stechsystem nach Anspruch 16, **dadurch gekennzeichnet, dass** der Positionsdetektor (28) die Position der durch das Vorspannmittel (16) gestrafften Haut (22) über das Kontaktglied (36) und/oder das Stechorgan (14) als Fühler abtastet.

18. Stechsystem nach Anspruch 16 oder 17, **dadurch gekennzeichnet, dass** der Positionsdetektor (28) eine Kapazitäts- oder Leitfähigkeitsänderung oder eine Kraftänderung beim Abtasten der Haut (22) erfasst.

19. Stechsystem nach einem der Ansprüche 15 bis 18, **dadurch gekennzeichnet, dass** das Stechelement (10) an eine vorzugsweise inkremental arbeitende Wegmesseinheit (50) zur Erfassung der Relativposition von Kontaktglied (36) und Stechorgan (14) angeschlossen ist.

20. Stechsystem nach einem der Ansprüche 14 bis 18, **dadurch gekennzeichnet, dass** der Stechantrieb (26) eine Einrichtung (30) zur Einstellung der Stechtiefe des Stechorgans (14) bezüglich einer erfassten Referenzposition der gestrafften Haut (22) aufweist.

21. Verfahren zur Hautdetektion für die Probennahme von Körperflüssigkeit, bei welchem die Position der Haut bezüglich einer Bewegungsachse eines Stechelements (10) erfasst wird, **dadurch gekennzeichnet, dass** die Haut (22) durch ein Vorspannmittel (16) des Stechelements (10) bei der Positionserfassung lokal gestrafft wird.

22. Verfahren nach Anspruch 20, **dadurch gekennzeichnet, dass** das Stechelement vor einer Stechbewegung nach der Positionserfassung in eine von der Haut (22) zurückgezogene Stellung bewegt wird.

23. Verfahren nach Anspruch 20 oder 21, **dadurch gekennzeichnet, dass** die Stechbewegung des Stechelements (10) zur Einstellung einer definierten Stechtiefe nach Maßgabe der erfassten Hautposition gesteuert wird.
